# EUROPEAN PATENT APPLICATION

(11) **EP 1 992 609 A1**
(43) Date of publication of application: **19.11.2008**
(21) Application number: 08008415.5
(22) Date of filing: 05.05.2008
(51) Int. Cl.: C07C 229/08, C12P 41/00

(54) **A process for the preparation of a (S)(+)-3-(aminomethyl)-5-methylhexanoic acid**

(30) Priority: 14.05.2007 IT MI20070971; 05.09.2007 IT MI20071722; 28.02.2008 IT MI20080318
(71) Applicant: Dipharma Francis S.r.l., 20021 Baranzate di Bollate MI (IT)
(72) Inventor: Riva, Sergio, 20030 Seveso (MI) (IT); Allegrini, Pietro, 20097 San Donato Milanese (MI) (IT); Serafini, Elena, 27100 Pavia (PV) (IT); Razzetti, Gabriele, 43100 Sesto San Giovanni (MI) (IT); Mantegazza, Simone, 20144 Milano (MI) (IT); Pastorello, Dario, 93012 Gela (CL) (IT)
(74) Representative: Minoja, Fabrizio

(57) **Abstract**

A process for the preparation of (S)(+)-3-(aminomethyl)-5-methylhexanoic acid (pregabalin) of formula (I) or a salt thereof, 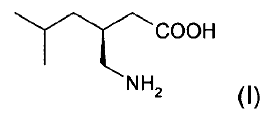 comprising the reaction of a compound of formula (II) 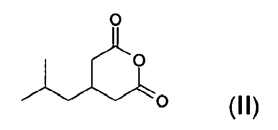 with an alcohol ROH, in the presence or absence of enzyme, to give a compound of formula (III) as herein defined 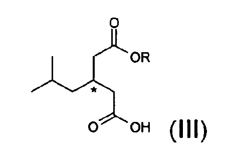 the transformation of a compound of formula (III) into a compound of formula (**VI**) or (**VIII**) as herein defined, 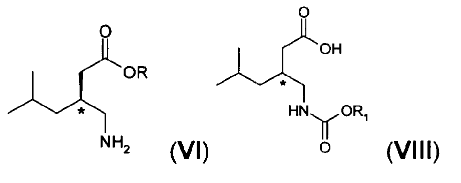 and the subsequent hydrolysis of a compound of formula (**VI**) or (**VIII**), to give pregabalin.

## Description

### FIELD OF THE INVENTION

The present invention relates to a novel process for the preparation of pregabalin, i.e. (*S*)(+)-3-(aminomethyl)-5-methylhexanoic acid, of formula (**I**)

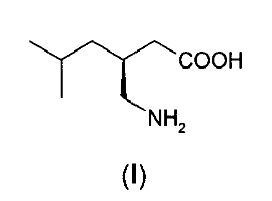

### TECHNOLOGICAL BACKGROUND

Pregabalin, disclosed in US 6,197,819, is used in the treatment of peripheral neuropathic pain, epilepsy and generalized anxiety disorder. US 5,637,767 discloses its preparation by conventional resolution of 3-aminomethyl-5-methylhexanoic acid racemic by formation of diastereomeric salts with homochiral acids or bases, separation of the diastereomeric pair by fractional crystallization or chromatography, followed by hydrolysis of the salt. This technique involves problems concerning production times and costs, especially if the undesired enantiomer cannot be racemized. US 6,359,169 discloses the preparation of pregabalin through an enantioselective reaction using a chiral auxiliary, e.g. Evans' oxazolidone (4R, 5S)-4-methyl-5-phenyl-2-oxazolidinone, which allows to carry out an asymmetric alkylation for introducing the desired stereocenter. After such asymmetric alkylation, which is usually carried out at cryogenic temperatures, the comparatively expensive chiral auxiliary has to be removed, which involves higher costs and longer production times. US 2005/0283023 discloses the preparation of pregabalin by enzymatic kinetic resolution of a cyano-diester according to the following scheme

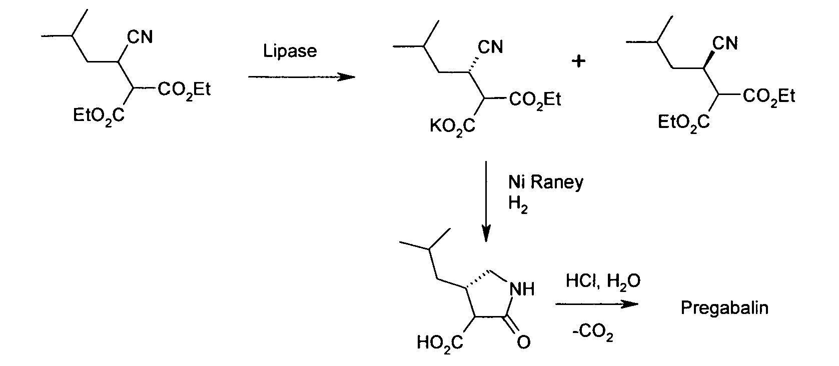

The above reported process is commercially feasible, but has some evident drawbacks: the non-hydrolized enantiomer has in fact to be recycled through a racemization step and the hydrogenation step is known to be problematic.

Organic Process Research & Development 1997; 1: 26-38, reports the synthesis of pregabalin, according to the scheme shown below, starting from a non-chiral diester by enzymatic hydrolysis with PLE (Porcine Liver Esterase) in water and DMSO at pH=8, to obtain the chiral monoester in 98% yield and 85% enantiomeric purity (the predominant enantiomer is the S one). The monoester is then converted to the respective monoamide in the presence of lithium methoxide and formamide, which monoamide undergoes Hofmann rearrangement to yield pregabalin.

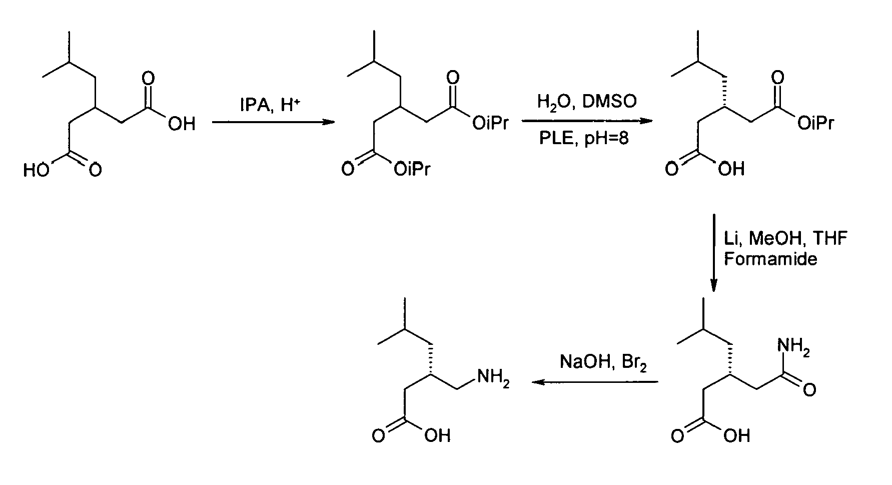

The enzymatic resolution is dramatically affected by the concentration of the reagents, in that it is selective only at low concentrations, which remarkably restricts application on a large scale.

It has now been found a process for the preparation of pregabalin, which overcomes the drawbacks of the prior art processes. This process makes use of an intermediate of formula (**III**), obtainable as an individual enantiomer or as a racemate, from an achiral compound of formula (**II**), which is then converted to pregabalin. Furthermore, this process uses inexpensive reagents and does not require dedicated apparatuses such as cryogenic reactors or high pressure hydrogenators, which are instead necessary in the known processes.

### DETAILED DISCLOSURE OF THE INVENTION

An object of the invention is a process for the preparation of (S)(+)-3-(aminomethyl)-5-methylhexanoic acid of formula (**I**) or a salt thereof, 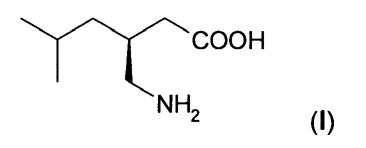 comprising:
a) the reaction of an achiral compound of formula (**II**) 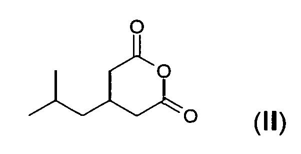 with an alcohol of formula ROH, wherein R is C₁-C₁₀ alkyl or aryl-C₁-C₆ alkyl, to obtain a 3-isobutyl glutaric acid ester of formula (**III**) or a salt thereof, as a mixture of the two enantiomers; or the reaction of an achiral compound of formula **(II)** with an alcohol of formula ROH, as defined above, in the presence of an enzyme, to obtain a 3-isobutyl glutaric acid ester of formula (**III**) or a salt thereof, in the form of the (*R*) or (*S*) enantiomer 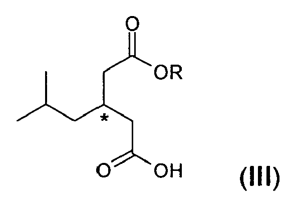 wherein R is as defined above and the asterisk * indicates the presence of a stereogenic carbon; and, when a compound of formula **(III)** is obtained as the (*S*) enantiomer, its conversion to another compound of formula **(III)** as the (*R*) enantiomer, wherein the meaning of R, being as defined above, is different from that of the starting compound;
b) the conversion of a compound of formula (**III**), as the (*R*) enantiomer, by rearrangement via formation of nitrene/isocyanate, in an aqueous acid solvent, to a compound of formula **(VI)** as the (*S*) enantiomer, 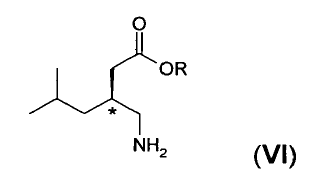 wherein R and the asterisk * are as defined above; or,
c) the conversion of a compound of formula **(III),** as the (*R*) enantiomer or as a mixture of the two enantiomers, by rearrangement *via* formation of nitrene/isocyanate, in a solvent of formula R₁-OH, wherein R₁ is an optionally substituted C₁-C₆ alkyl, aryl or aryl-C₁-C₆ alkyl group, to give respectively a compound of formula **(VII),** as the (*S*) enantiomer, or as a mixture of the two enantiomers, 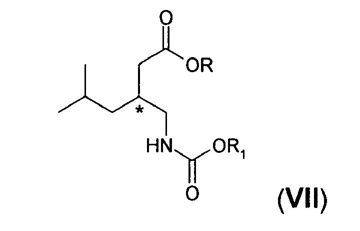 wherein R, R₁ and the asterisk * are as defined above;
d) the hydrolysis of a compound of formula **(VII),** to give a compound of formula **(VIII)** as the (*S*) enantiomer or a mixture of the two enantiomers, 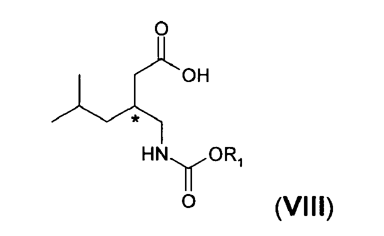 wherein R₁ and the asterisk * are as defined above; and, if desired, its enantiomeric enrichment in the (*S*) enantiomer; and
e) the hydrolysis of the (*S*) enantiomer of a compound of formula **(VI)** or **(VIII),** respectively as obtained from steps b) and d), to give a compound of formula (**I**) or a salt thereof; and, if desired, the conversion of a compound of formula (**I**) to a salt thereof, or vice versa.

R as a C₁-C₁₀ alkyl group is preferably a C₁-C₄ alkyl group, for example methyl, ethyl, propyl, butyl, isobutyl, or tert-butyl; in particular methyl or ethyl.

R as an aryl-C₁-C₆ alkyl group is for example phenyl-C₁-C₆ alkyl or naphthyl-C₁-C₆ alkyl, preferably phenyl-C₁-C₄ alkyl, most preferably benzyl or phenylethyl.

R₁ as a C₁-C₆ alkyl group is optionally substituted with 1 to 5 substituents, preferably 1 or 2, independently selected from halogen, cyano and C₁-C₆ dialkyl-amino, for example dimethyl-, diethyl-, or diisopropyl-amino. R₁ is preferably a C₁-C₄ alkyl group, more preferably methyl, ethyl, propyl, i-propyl or tert-butyl; most preferably methyl, ethyl or I-propyl.

R₁ as an aryl group is optionally substituted with 1 to 5 substituents, preferably 1, 2 or 3, independently selected from C₁-C₆ dialkyl-amino, nitro, cyano and halogen. R₁ is for example phenyl or naphthyl; most preferably phenyl.

R₁ as an aryl-C₁-C₆ alkyl group is optionally substituted at the aryl moiety and/or at the alkyl moiety with 1 to 5, preferably 1 or 2, substituents independently selected from halogen, nitro, cyano and C₁-C₆ dialkyl-amino, for example dimethyl-, diethyl-, or diisopropyl-amino. R₁ is for example phenyl-C₁-C₆ alkyl or naphthyl-C₁-C₆ alkyl, preferably phenyl-C₁-C₄ alkyl, most preferably benzyl or phenylethyl.

An alkyl group or residue can be straight or branched.

A halogen is for example chlorine, fluorine, bromine or iodine, in particular chlorine and bromine.

An enzyme according to step a) can be a hydrolase, selected for example from the following groups x) and y):
x) lipase from *Candida rugosa;*
y) comprising CAL B lipase (from *Candida antarctica),* porcine pancreas lipase, chymotrypsin, PS lipase (from *Pseudomonas),* lipase CV (from *Chromobacterium viscosum*), lipase from *Candida cylindracea*, lipase A (from *Aspergillus),* lipase CE-5 (from *Humicola lanuginosa),* esterase from porcine liver and protease (*subtilisin Carlsberg);* preferably CAL B lipase (from *Candida antarctica).*

When the enzyme used is lipase from *Candida rugosa,* the compound of formula **(III)** is obtained as the (*R*) enantiomer.

When an enzyme belonging to group y) is used, a compound of formula (**III**) is obtained as the (*S*) enantiomer.

The enzyme can be either free or immobilized, purified or in a matrix, preferably immobilized.

According to the invention, an enzyme unit "U" is defined as the amount of enzyme which catalyzes the release of 1.0 µmol of fatty acid which can be titred per minute, from the corresponding triglyceride at 30°C and pH= 7.

The ratio of enzyme to substrate of formula (**II**) can approximately range from 1.0 to 10.0 U per mg of substrate, preferably approximately from 1.0 U to 3.0 U per mg of substrate.

A compound of formula **(III)** obtained according to step a) in the presence of an enzyme, has enantiomeric purity approximately ranging from 60% to 95%. Said purity can optionally be increased up to 99.5% by techniques known to those skilled in the art, for example by crystallization.

The reaction between a compound of formula **(II)** and an alcohol ROH, according to step a), is preferably carried out using the alcohol itself as the solvent. Alternatively, the reaction can optionally be carried out in a solvent, selected for example from a dipolar aprotic solvent, typically dimethylformamide, dimethylacetamide, acetonitrile, dimethylsulfoxide; a ketone, typically acetone or methyl isobutyl ketone; an ether, typically tetrahydrofuran, methyl-tert-butyl ether or dioxane; a chlorinated solvent, typically dichloromethane; a tertiary alcohol, e.g. tert-butyl alcohol, teramyl alcohol; or an apolar solvent, typically toluene or hexane; or a mixture of two or more, preferably of two or three, of said solvents. In this case, the reaction is preferably carried out in methyl-tert-butyl ether or in ter-amyl alcohol.

When the reaction between a compound of formula (**II**) and an alcohol ROH, according to step a), is carried out in the absence of enzyme, it can be optionally carried out in the presence of an achiral organic base, e.g. a C₁-C₄ trialkylamine, typically methyl-diethylamine, triethylamine, tributylamine or ethyl-diisopropylamine; in particular triethylamine.

The reaction can be carried out at a temperature approximately ranging from 15 to 45°C, preferably approximately from 20 to 30°C. Reaction times can approximately range between 20 min and 48 h.

When the reaction of step a) is carried out in the presence of a solvent, the amount of alcohol of formula ROH used can approximately range from 0.8 to 3 mols per mole of substrate of formula **(II),** preferably approximately from 1.1 to 2.1.

The conversion of a compound of formula **(III),** as the (*S*) enantiomer or a salt thereof, to another compound of formula **(III),** as the (*R*) enantiomer or a salt thereof, can be obtained by means of a process comprising the esterification of the carboxylic group in a compound of formula (**III**) to give an ester of formula **(IIIa)** 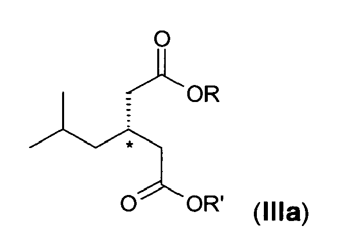 wherein the asterisk * is as defined above, and R and R', different from each other, have the same meanings as R defined above; the subsequent selective basic hydrolysis of the ester group COOR in the compound of formula **(IIIa);** and, if desired, the conversion of a compound of formula (**III**) to a salt thereof.

In a compound of formula (**IIIa**), preferably R is methyl or ethyl, and R' is tert-butyl.

The esterification of the carboxylic group in a compound of formula **(III)** to give a compound of formula **(IIIa),** the subsequent basic hydrolysis selective of the group COOR and the optional salification can be carried out according to known methods. By way of example, a compound of formula **(IIIa)** wherein R' is tert-butyl can be obtained by treating a compound of formula (**III**) in the presence of tert-butyl alcohol and a condensing agent, e.g. dicyclohexylcarbodiimide. The selective basic hydrolysis of the COOR group can be carried out for example by treating a compound of formula **(IIIa)** with an alkali base in an aqueous or hydroalcoholic solvent.

The conversion of a compound of formula **(III),** as the (*R*) enantiomer, by rearrangement via formation of nitrene/isocyanate, to obtain a compound of formula **(VI)** as the (*S*) enantiomer, can be carried out, for example, according to the Curtius, Schmidt, Lossen or Hofmann reactions, following known methods.

For example, the Curtius reaction can be carried out by reaction of a compound of formula **(III)** as the (*R*) enantiomer, with a halogenating agent, preferably thionyl chloride or oxalyl chloride, and subsequent treatment with sodium azide or directly with diphenylphosphoryl azide, in the presence of an organic base, in particular triethylamine, diisopropylethylamine or pyridine, to obtain the respective acyl-azide. The acyl-azide is converted by heating to the corresponding isocyanate which spontaneously converts to a compound of formula (**VII**) as the (*S*) enantiomer, in the presence of an aqueous acid solvent.

The reaction can be carried out according to known methods, for example at a temperature approximately ranging from 10 to 100°C, preferably from 50 to 90°C, for a time ranging between 2 and 15 h, preferably between 5 and 10 h.

An acid aqueous solvent is typically an aqueous solution of a mineral acid, e.g. sulfuric acid or hydrochloric acid, preferably hydrochloric acid.

The conversion of a compound of formula **(III),** as the (*R*) enantiomer or as a mixture of the two enantiomers, by rearrangement *via* formation of nitrene/isocyanate to obtain a compound of formula **(VII)** respectively, as the (S) enantiomer or a mixture of the two enantiomers, can be carried out for example according to the Schmidt, Lossen, Hofmann or Curtius reactions.

The Schmidt reaction can be carried out according to known methods, for example by treating a compound of formula (**III**) with hydrazoic acid in the presence of sulfuric acid. Alternatively, the rearrangement can be carried out according to the Lossen reaction, by reacting a compound of formula (**III**) with a halogenating agent, preferably thionyl chloride or oxalyl chloride. Subsequently, the corresponding acylated hydroxamic acid is obtained by reaction with an acyl-hydroxylamine, preferably acetyl-hydroxylamine. Treatment of the hydroxamic acid with an alkali hydroxide yields the isocyanate, which spontaneously converts to a compound of formula **(VII)** in the presence of a solvent of formula R₁-OH.

According to the Hofmann reaction, the carboxylic acid of formula **(III)** is transformed into the corresponding amide according to known methods, and the latter is then treated with an alkali hypohalide, preferably sodium hypochlorite, to obtain the isocyanate, which spontaneously converts to a compound of formula **(VII)** in the presence of a solvent of formula R₁-OH.

According to the Curtius reaction, a compound of formula **(III)** can be reacted with a halogenating agent, preferably thionyl chloride or oxalyl chloride, and subsequently treated with sodium azide or directly with diphenylphosphoryl azide, in the presence of an organic base, in particular triethylamine, diisopropylethylamine or pyridine, to obtain the corresponding acyl-azide. The latter is heated to obtain the corresponding isocyanate, which spontaneously converts to a compound of formula **(VII)** in the presence of a solvent of formula R₁-OH. The reaction can be carried out according to known methods, for example at a temperature approximately ranging from 10 to 100°C, preferably from 50 to 90°C, for a time ranging between 2 and 15 h, preferably between 5 and 10 h.

The hydrolysis of a compound of formula **(VII),** as a mixture of the two enantiomers, to give a compound of formula **(VIII),** as a mixture of the two enantiomers, is preferably a basic hydrolysis that can be carried out according to known methods, for example by reaction in water in the presence of an alkali or alkaline-earth base, typically sodium hydroxide or potassium hydroxide.

The subsequent enantiomeric enrichment of a compound of formula **(VIII)** in its (*S*) enantiomer can be obtained through formation of a diastereomeric salt thereof with a chiral base, followed by separation of diastereomeric pair, and subsequent hydrolysis of the resulting diastereomeric salt of the compound of formula (**VIII**) (*S*) enantiomer, according to known methods.

A chiral base can be a chiral amine, selected e.g. from those reported in *"*S.H. Wilen - Tables of Resolving Agents and Optical Resolutions Un. of Notre Dame Press*",* for example S-(-)-phenylethylamine, S-(-)-naphthylethylamine, brucine and quinine, preferably S-(-)-phenylethylamine.

The hydrolysis of the (*S*) enantiomer of a compound of formula **(VI)** or **(VIII),** according to step e), to give a compound of formula **(I),** is typically an acid hydrolysis and can be carried out, for example, by treatment with a mineral acid, typically hydrochloric acid, according to known methods.

In the present invention, "enantiomeric mixture" means a substantially 50% mixture of the two enantiomers.

In the present invention, "(*R*) or (*S*) enantiomer" means a compound as the individual (*R*) or (*S*) enantiomer, or a mixture respectively enriched in the (*R*) or (*S*) enantiomer.

"Individual (*R*) or (*S*) enantiomer" means that the enantiomeric purity is at least equal to or higher than 96%, preferably at least around 99%.

"Mixture enriched in the (*R*) or (*S*) enantiomer" means that the enantiomeric purity is at least equal to or higher than 51%, preferably at least about 99%.

The enantiomeric purity is defined as S/(S+R)x100, wherein S and R are respectively the amount of the (*S*) and (*R*) enantiomers.

In the present invention, the term "compound of formula **(I) - (VIII)"** means the compound as it is or a salt thereof, typically a pharmaceutically acceptable salt. Said salt is in particular a salt with a pharmaceutically acceptable cation or anion, for example a lithium, sodium, potassium, magnesium or aluminium salt, or a chloride, bromide, sulfate or camphforsulfonate. Both salification and cleavage of the salt can be carried out according to known methods.

The resulting pregabalin has enantiomeric purity equal to or higher than about 98%, preferably equal to or higher than 99%, more preferably higher than 99.5%, most preferably higher than 99.9%, which fulfils the regulatory requirements for medicaments. Pregabalin with said enantiomeric purity degree is novel and is a further object of the invention.

Pregabalin obtained according to the process of the invention has mean particle size D50 ranging from 10 to 250 micrometres, which can be further reduced, for example by a fine grinding process according to known techniques.

Pregabalin crystalline form obtained according to the process herein described is the same as described in CN 1634869A.

The compounds of formula **(VII)** and **(VIII),** both as individual enantiomers (*S*) or (*R*), and as mixtures thereof, and the salts thereof, are novel and are a further object of the invention.

Specific examples of compounds of formula **(VII)** are:
- Methyl (*S*)-{4-methyl-2-[(1-methoxycarbonyl)-methyl-]-pentyl-}-carbamate,
- Methyl (*S*)-{4-methyl-2-[(1-ethoxycarbonyl)-methyl-]-pentyl-}-carbamate,
- Methyl (*S*)-{4-methyl-2-[(1-propoxycarbonyl)-methyl-]-pentyl-}-carbamate,
- Methyl (*S*)-{4-methyl-2-[(1-butoxycarbonyl)-methyl-]-pentyl-}-carbamate,
- Methyl (*S*)-{4-methyl-2-[(1-isobutyloxycarbonyl)-methyl-]-pentyl-}-carbamate,
- Methyl (*S*)-{4-methyl-2-[(1-octyloxycarbonyl)-methyl-]-pentyl-}-carbamate,
- Ethyl (*S*)-{4-methyl-2-[(1-methoxycarbonyl)-methyl-]-pentyl-}-carbamate,
- Ethyl (*S*)-{4-methyl-2-[(1-ethoxycarbonyl)-methyl-]-pentyl-}-carbamate,
- Ethyl (*S*)-{4-methyl-2-[(1-propoxycarbonyl)-methyl-]-pentyl-}-carbamate,
- Ethyl (*S*)-{4-methyl-2-[(1-butoxycarbonyl)-methyl-]-pentyl-}-carbamate,
- Ethyl (*S*)-{4-methyl-2-[(1-isobutyloxycarbonyl)-methyl-]-pentyl-}-carbamate,
- Ethyl (*S*)-{4-methyl-2-[(1-octyloxycarbonyl)-methyl-]-pentyl-}-carbamate,
- Isopropyl (*S*)-{4-methyl-2-[(1-methoxycarbonyl)-methyl-]-pentyl-}-carbamate,
- Isopropyl (*S*)-{4-methyl-2-[(1-ethoxycarbonyl)-methyl-]-pentyl-}-carbamate,
- Isopropyl (*S*)-{4-methyl-2-[(1-propoxycarbonyl)-methyl-]-pentyl-}-carbamate,
- Isopropyl (*S*)-{4-methyl-2-[(1-butoxycarbonyl)-methyl-]-pentyl-}-carbamate,
- Isopropyl (*S*)-{4-methyl-2-[(1-isobutyloxycarbonyl)-methyl-]-pentyl-}-carbamate, and
- Isopropyl (*S*)-{4-methyl-2-[(1-octyloxycarbonyl)-methyl-]-pentyl-}-carbamate;

in particular as (*S*) enantiomers, or as a mixture of enantiomers and the salts thereof.

Specific examples of compounds of formula **(VIII),** both as (*R*) or (*S*) enantiomers, in particular as (*S*) enantiomers and the salts thereof, in particular with a chiral base, preferably with S-(-)-phenylethylamine, are:
Methyl {4-methyl-2-[(1-carboxy)-methyl]-pentyl}-carbamate,
Ethyl {4-methyl-2-[(1-carboxy)-methyl]-pentyl}-carbamate, and
Isopropyl {4-methyl-2-[(1-carboxy)-methyl]-pentyl}-carbamate.

The following examples illustrate the invention.

### EXAMPLE 1 - SYNTHESIS OF METHYL (S)-3-ISOBUTYL-GLUTARATE (III)

A 1 L three-necked round-bottom flask, under nitrogen atmosphere, is loaded with 3-isobutylglutaric anhydride (5.0 g; 29 mmol) of formula **(II)** and methyl isobutyl ether (MTBE) (100 ml). The resulting solution, kept at temperatures of about 20-25°C, is added with methanol (2.5 g) and CAL-B Novozym ® 435 (2.5 g), and the solution is kept under stirring for about 3 h. After that, the enzyme is filtered off and the solvent is evaporated off. The resulting product has 85% enantiomeric purity.

Following similar procedures, the following compounds are obtained:
Ethyl (*S*)-3-isobutyl-glutarate,
Propyl (*S*)-3-isobutyl-glutarate,
Isobutyl (*S*)-3-isobutyl-glutarate, and
Octyl (*S*)-3-isobutyl-glutarate.

### EXAMPLE 2 - SYNTHESIS OF METHYL (R)-3-ISOBUTYL-GLUTARATE (III)

A 1 L three-necked round-bottom flask, under nitrogen atmosphere, is loaded with 3-isobutylglutaric anhydride (5.0 g; 29 mmol) of formula **(II)** and MTBE (100 ml). The resulting solution, is kept at temperatures of about 20-25°C, and added with methanol (2.5 g) and lipase from *Candida rugosa* (2.5 g), and the solution is kept under stirring for about 3 h. After that, the enzyme is filtered off and the solvent is evaporated off. The resulting product has 67% enantiomeric purity.

Following similar procedures, the following compounds are obtained:
Ethyl (*R*)-3-isobutyl-glutarate,
Propyl (*R*)-3-isobutyl-glutarate,
Isobutyl (*R*)-3-isobutyl-glutarate, and
Octyl (*R*)-3-isobutyl-glutarate.

### EXAMPLE 3 - SYNTHESIS OF METHYL 3-ISOBUTYL-GLUTARATE (III)

A 1 L three-necked round-bottom flask, under nitrogen atmosphere, is loaded with 3-isobutylglutaric anhydride (50.0 g; 0.290 mol) of formula **(II),** methanol (500 ml) and triethylamine (29.3 g; 0.290 mol); the resulting solution is kept under stirring at room temperature for about 16-18 h. After completion of the reaction, the solvent is evaporated off, the mixture is taken up with water (200 ml), acidified to pH 3-4 with 37% hydrochloric acid and extracted with toluene (3x150 ml). The combined organic phases are evaporated under reduced pressure, to obtain a pale yellow oil (58.6 g; yield: 95%).

¹H-NMR (300 MHz, CDCl₃, 28°C): *δ 3.65 (s, 3H); 2.40 (m, 5H); 1.60 (m, 1H); 1.20 (m, 2H); 0.90 (d, 6H).*

Following similar procedures, the following compounds are obtained:
Ethyl 3-isobutyl-glutarate,
Propyl 3-isobutyl-glutarate,
Isobutyl 3-isobutyl-glutarate, and
Octyl 3-isobutyl-glutarate.

### EXAMPLE 4 - SYNTHESIS OF METHYL TERT-BUTYL (R)-3-ISOBUTYL-GLUTARATE (IIIA)

A 100 ml three-necked round-bottom flask, under nitrogen atmosphere, is added at room temperature with: methyl (*S*)-3-isobutylglutarate (24.7 mmol; 5.00 g) of formula (**III**), tert-butanol (25.9 mmol; 1.90 g), dimethylaminopyridine (4.90 mmol; 0.610 g) and dicyclohexylcarbodiimide (27.2 mmol; 5.70 g) in dichloromethane (50 ml). The mixture is reacted for about 5 h, then the solid is filtered off. The filtered solution is concentrated to small volume to obtain 6.06 g of an oil, in 95% yield.

¹H-NMR (300 MHz, CDCl₃, 28°C): *δ 3.6 (s, 3H); 2.4-2.2 (m, 5H); 1.6 (m, 1H); 1.4 (s, 9H); 1.2 (m, 2H); 0.9 (d, 6H).*

### EXAMPLE 5 - SYNTHESIS OF TERT-BUTYL (R)-3-ISOBUTYL-GLUTARATE (III)

A 25 ml round-bottom flask, under nitrogen atmosphere, is added with: methyl tert-butyl (*R*)-3-isobutylglutarate (11.6 mmol; 3.00 g) of formula **(IIIa),** sodium hydroxide flakes (17.4 mmol; 0.700 g) in water (10 ml) and the mixture is left under stirring at room temperature for about 18 h. The aqueous solution is acidified to pH below 2 with hydrochloric acid and extracted with toluene (3x10 ml). The separated organic phase is dried over sodium sulfate, filtered and concentrated to small volume under reduced pressure, to obtain 2.6 g of a pale yellow oil, in 94% yield.

¹H-NMR (300 MHz, CDCl₃, 28°C): *δ 2.45-2.20 (m, 5H); 1.65 (m, 1H); 1.45 (s, 9H); 1.20 (m, 2H); 0.90 (d, 6H).*

### EXAMPLE 6 - SYNTHESIS OF METHYL (S)-(+)-3-AMINOMETHYL-5-METHYL-HEXANOATE (VI)

A 50 ml three-necked round-bottom flask, under nitrogen atmosphere, is added with methyl (*R*)-3-isobutylglutarate (3.4 g; 17 mmol) of formula **(III),** and a solution of triethylamine (1.9 g; 19 mmol) in water (25 ml). The solution is heated to a temperature of about 80-85°C and added with diphenylphosphoryl azide (2.2 g; 18 mmol) in about 2 h; after completion of the addition the mixture is refluxed for about 1 h. After that, the reaction mixture is cooled to room temperature and diluted with a sodium bicarbonate 5% w/w solution (30 ml); extracted with ethyl acetate (80 ml) and the solvent is evaporated off to dryness, to obtain the title compound.
GC-MS (M+·): 173

### EXAMPLE 7 - SYNTHESIS OF METHYL (S)-{4-METHYL-2-[(1-METHOXYCARBONYL)-METHYL-]-PENTYL-}-CARBAMATE (VII)

A 50 ml three-necked round-bottom flask, under nitrogen atmosphere, is added with methyl (*R*)-3-isobutylglutarate (3.4 g; 17 mmol) of formula **(III),** and a solution of triethylamine (1.9 g; 19 mmol) in methanol (25 ml). The solution is refluxed and added with diphenylphosphoryl azide (2.2 g; 18 mmol) in about 2 h; after completion of the addition the mixture is refluxed for about 1 h. The reaction mixture is cooled to room temperature, poured in a sodium hydroxide 10% w/w solution (30 ml), extracted with toluene (3 x 25 ml), and the combined organic phases are washed with water (2 x 25 ml). The washed organic phase is concentrated under reduced pressure, to obtain 3.5 g of a pale yellow oil; 80% yield.

¹H-NMR (300 MHz, CDCl₃, 28°C): *δ 3.6* (s, *6H); 3.2 (m, 1H); 3.0 (m, 1H);* 2.3 *(m, 2H);* 2.1 *(m, 1H); 1.6 (m, 1H); 1.2 (m, 2H); 0.9 (m, 6H).*
GC-MS (M+): 231

Following similar procedures, the following compound is obtained:
Isopropyl (*S*)-{4-methyl-2-[(1-methoxycarbonyl)-methyl-]-pentyl-}-carbamate.

### EXAMPLE 8 - SYNTHESIS OF METHYL {4-METHYL-2-[(1-METHOXYCARBONYL)-METHYL-]-PENTYL-}-CARBAMATE (VII)

A 50 ml three-necked round-bottom flask, under nitrogen atmosphere, is added with methyl 3-isobutylglutarate (3.4 g; 17 mmol) of formula **(III),** and a solution of triethylamine (1.9 g; 19 mmol) in methanol (25 ml). The solution is refluxed and added with diphenylphosphoryl azide (2.2 g; 18 mmol) in about 2-3 h; after completion of the addition the mixture is refluxed for about 1 h. The reaction mixture is then cooled to room temperature, poured in a sodium hydroxide 10% w/w solution (30 ml), extracted with toluene (3 x 25 ml) and the combined organic phases are washed with water (2 x 25 ml). The washed organic phase is concentrated under reduced pressure, to obtain 3.5 g of a pale yellow oil; 80% yield.

¹H-NMR (300 MHz, CDCl₃, 28°C): *δ* 3.6 *(s, 6H);* 3.2 *(m, 1H); 3.0 (m, 1H);* 2.3 *(m, 2H);* 2.1 *(m, 1H);* 1.6 *(m, 1H);* 1.2 *(m, 2H); 0.9 (m, 6H).*
GC-MS (M+): 231

Following similar procedures, the following compound is obtained:
Isopropyl {4-methyl-2-[(1-methoxycarbonyl)-methyl-]-pentyl-}-carbamate.

### EXAMPLE 9 - SYNTHESIS OF ISOPROPYL {4-METHYL-2-[(1-CARBOXY)-METHYL-]-PENTYL-}-CARBAMATE (VIII)

A 50 ml three-necked round-bottom flask, under nitrogen atmosphere, is added with isopropyl {4-methyl-2-[(1-methoxycarbonyl-)-methyl-]-pentyl-}-carbamate (62.0 g; 0.237 mmol) of formula **(VII)** ad a sodium hydroxide solution (16.0 g; 0.400 mol) in water (300 ml) and is kept under stirring at room temperature for 18 h. A 37% w/w hydrochloric acid solution is added to acid pH (60 ml); the aqueous phase is extracted with toluene (3 x 300 ml). The organic phase is concentrated to small volume, to obtain a pale yellow oil (56.9 g; yield: 97%).

¹H-NMR (300 MHz, CDCl₃, 28°C): *δ 7.00 (broad, 1H exchangeable with D₂O); 4. 70 (m, 1H); 3.00 (m, 1H); 2. 80 (m, 1H); 2.10 (m, 2H); 1.95 (m, 1H); 1.60 (m, 1H); 1.20-1.00 (m, 8H); 0.80 (d, 6H).*

Following similar procedures, the following compounds are obtained:
Methyl {4-methyl-2-[(1-carboxy)-methyl]-pentyl}-carbamate, and Ethyl {4-methyl-2-[(1-carboxy)-methyl]-pentyl}-carbamate.

### EXAMPLE 10 - OPTICAL RESOLUTION OF ISOPROPYL (S)-{4-METHYL-2-[(1-CARBOXY)-METHYL-]-PENTYL-}-CARBAMATE (VIII)

A 100 ml three-necked round-bottom flask, under nitrogen atmosphere, is added with isopropyl {4-methyl-2-[(1-carboxy)-methyl-]-pentyl-}-carbamate (13.0 g, 53.0 mmol) of formula **(VIII),** triethylamine (2.70 g; 26.5 mmol) and (*S*)-(-)-phenylethylamine (3.2 g, 26.5 mmol) in a water/isopropanol 95:5 mixture (50 ml). The solution is heated to a temperature of about 60-65°C and slowly cooled to about 35°C, then heated again to 50°C and left to spontaneously cool to room temperature. The mixture is then cooled to 0-5°C for at least 1 h and the solid is filtered off and washed with cold water (2 x 10 ml), then with toluene (3 x 10 ml). The product is dried in a static dryer at 50°C under vacuum for 18 hours, to obtain 8.9 g of a product having a 98(*S*):2(*R*) enantiomeric ratio in a 92% yield.

Following similar procedures, the following compounds are obtained:
Methyl (*S*)-{4-methyl-2-[(1-carboxy)-methyl]-pentyl}-carbamate, and Ethyl (*S*)- 4-methyl-2-[(1-carboxy)-methyl]-pentyl}-carbamate.

### EXAMPLE 11 - SYNTHESIS OF (S)-(+)-3-AMINOMETHYL-5-METHYLHEXANOIC ACID (I)

A 50 ml three-necked round-bottom flask, under nitrogen atmosphere, is added with isopropyl (*S*)-{4-methyl-2-[(1-carboxy)-methyl-]-pentyl-}-carbamate (*S*)-(-)-phenylethylamine salt (60.0 g, 164 mmol), of formula **(VIII)** having a 98(S):2(R) enantiomeric ratio, and a 37% w/w hydrochloric acid solution (17.8 g, 180.4 mmol) in water (200 ml), and extracted with toluene (2 x 200 ml). The organic phase is concentrated to small volume, taken up with 30% w/w hydrochloric acid (59.6 g, 490 mmol) and the mixture is heated to 90-95°C for 24 h. The solution is cooled to 0-5°C, added with sodium hydroxide (18.0 g, 450 mmol) and kept under stirring while cooling for at least 1 h. The solid is filtered and washed with a water/isopropanol 8:2 solution cooled to 0-5°C (2 x 15 ml) and dried in a static dryer. A white solid is obtained, 21.1 g, with 99.96(S):0.04(R) enantiomeric ratio; 82% yield.

¹H-NMR (300 MHz, CDCl₃, 28°C): *δ* 2.95 (m, 2H); 2.30-2.05 (m, 3H); 1.60 (m, 1 H); 1.2 (m, 2H); 0.80 (dd, 6H).

### EXAMPLE 12 - SYNTHESIS OF (S)-(+)-3-AMINOMETHYL-5-METHYLHEXANOIC ACID SODIUM SALT (I)

A 50 ml three-necked round-bottom flask, under nitrogen atmosphere, is added with methyl (*S*)-3-aminomethyl-5-methyl-hexanoate (2.1 g, 12 mmol) of formula (**VI**) and a 37% w/w hydrochloric acid solution (20 ml); the resulting suspension is heated at a temperature of about 90-100°C for 18 h. A 50% w/w sodium hydroxide solution is added to basic pH (15 ml) and the solution is concentrated to small volume and diluted with methanol (25 ml). The suspended salts are filtered off and the resulting solution is concentrated under reduced pressure. 1.7 g of a product (88% yield) with enantiomeric purity higher than 99% are obtained.

### EXAMPLE 13 - SYNTHESIS (S)-(+)-3-AMINOMETHYL-5-METHYLHEXANOIC ACID (I)

A 500 ml three-necked round-bottom flask, under nitrogen atmosphere, is added with isopropyl (*S*)-{4-methyl-2-[(1-methoxycarbonyl-)-methyl-]-pentyl-}-carbamate (46.8 g; 190 mmol) of formula **(VII)** and 30% hydrochloric acid (57.8 g, 0.475 mol). The mixture is heated at a temperature of 90°C for 24-48h. After completion of the reaction, 41% aqueous monomethylamine (26.7 ml) is added to pH about 6 and the mixture is left to spontaneously cool to room temperature. The mixture is cooled to 0-5°C for at least 1 h, then the solid is filtered and washed with a water/isopropanol 1:1 mixture cooled to 0-5°C (3 x 15 ml). The solid is dried in a static dryer at 50-60°C for 16-18 h. 26.6 g of a white solid are obtained, having a 99.94(S):0.06(R) enantiomeric ratio, in an 88% yield.

¹H-NMR (300 MHz, CDCl₃, 28°C): *δ* 2.95 (m, 2H); 2.30-2.05 (m, 3H); 1.60 (m, 1 H); 1.2 (m, 2H); 0.80 (dd, 6H).

The resulting Pregabalin has 99.5% purity, and mean particle size D50 of about 50 micrometres.

### EXAMPLE 14 - SYNTHESIS OF METHYL 3-ISOBUTYL-GLUTARATE (III)

A 1 L three-necked round-bottom flask, under nitrogen atmosphere, is added with 3-isobutylglutaric anhydride (50.0 g; 0.290 mol) and methanol (500 ml). The resulting solution is kept under stirring at room temperature for about 16-18 h. After completion of the reaction, the solvent is evaporated off. The title product is obtained as a pale yellow oil, 59.8 g, in 97% yield.

¹H-NMR (300 MHz, CDCl₃, 28°C): *δ* 3.65 *(s, 3H); 2.40 (m, 5H); 1.60 (m, 1H); 1.20 (m, 2H); 0.90 (d, 6H).*

## Claims

1. A process for the preparation of (*S*)(+)-3-(aminomethyl)-5-methylhexanoic acid of formula **(I)** or a salt thereof, 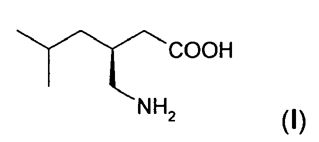 comprising:
a) the reaction of an achiral compound of formula **(II)** 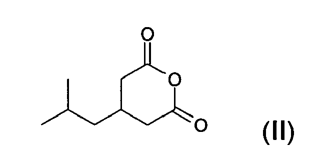 with an alcohol of formula ROH, wherein R is C₁-C₁₀ alkyl or aryl-C₁-C₆ alkyl, to obtain a 3-isobutyl glutaric acid ester of formula (**III**) or a salt thereof, as a mixture of the two enantiomers; or the reaction of an achiral compound of formula (**II**) with an alcohol of formula ROH, as defined above, in the presence of an enzyme, to obtain a 3-isobutyl glutaric acid ester of formula **(III)** or a salt thereof, as the (*R*) or (*S*) enantiomer 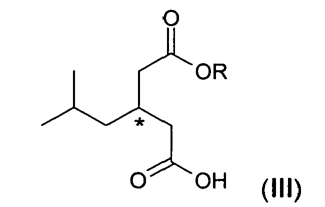 wherein R is as defined above and the asterisk * indicates the presence of a stereogenic carbon; and, when a compound of formula **(III)** is obtained as the (*S*) enantiomer, its conversion to another compound of formula **(III)** as the (*R*) enantiomer, wherein the meaning of R, being as defined above, is different from that of the starting compound;
b) the conversion of a compound of formula (**III**), as the (*R*) enantiomer, by rearrangement via formation of nitrene/isocyanate, in an aqueous acid solvent, to a compound of formula (**VI**) as the (*S*) enantiomer 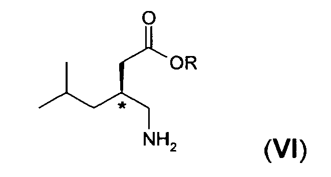 wherein R and the asterisk * are as defined above; or,
c) the conversion of a compound of formula **(III),** as the (*R*) enantiomer or as a mixture of the two enantiomers, by rearrangement via formation of nitrene/isocyanate in a solvent of formula R₁-OH, wherein R₁ is an optionally substituted C₁-C₆ alkyl, aryl or aryl-C₁-C₆ alkyl group, to respectively give a compound of formula **(VII)** as the (*S*) enantiomer, or as a mixture of the two enantiomers, 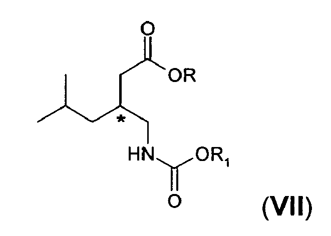 wherein R, R₁ and the asterisk * are as defined above;
d) the hydrolysis of a compound of formula **(VII)** to give a compound of formula **(VIII),** as the (*S*) enantiomer or a mixture of the two enantiomers, 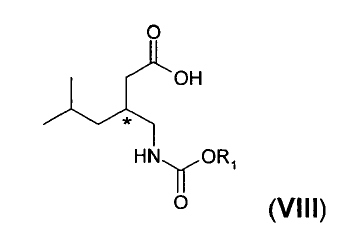 wherein R₁ and the asterisk * are as defined above; and, if desired, its enantiomeric enrichment in the (*S*) enantiomer; and
e) the hydrolysis of the (*S*) enantiomer of a compound of formula **(VI)** or **(VIII),** as obtained respectively at steps b) and d), to give a compound of formula (**I**) or a salt thereof; and, if desired, the conversion of a compound of formula (**I**) to a salt thereof, or *vice versa.*

2. The process as claimed in claim 1, wherein the enzyme is a hydrolase.

3. The process as claimed in claim 2, wherein the hydrolase is a lipase from *Candida rugosa,* CAL B lipase (from *Candida antarctica),* lipase from porcine pancreas, chymotrypsin, lipase PS (from *Pseudomonas),* lipase CV (from *Chromobacterium viscosum),* lipase from *Candida cylindracea,* lipase A (from *Aspergillus),* lipase CE-5 (from *Humicola lanuginosa),* esterase from porcine liver or protease (*subtilisin Carlsberg).*

4. The process as claimed in claim 3, wherein the hydrolase is a lipase from *Candida rugosa* or CAL B lipase (from *Candida antarctica*).

5. The process as claimed in claim 1, step a), wherein the reaction between a compound of formula **(II)** and an alcohol ROH is carried out in the absence of an enzyme.

6. The process as claimed in claim 1, wherein the conversion of a compound of formula **(III),** as the (*S*) enantiomer or a salt thereof, to another compound of formula (**III**), as the (*R*) enantiomer or a salt thereof, is obtained by a process comprising the esterification of the carboxylic group in a compound of formula **(III)** to give an ester of formula **(IIIa)** 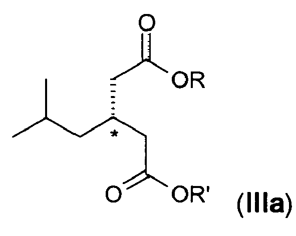 wherein the asterisk * is as defined in claim 1, and R and R', different from each other, have the same meanings as R as defined in claim 1; the subsequent selective basic hydrolysis of the ester group COOR in the compound of formula (**IIIa**); and, if desired, the conversion of a compound of formula **(III)** to a salt thereof.

7. A compound of formula **(VII),** both as the (*S*) or (*R*) enantiomer, and as mixtures thereof or a salt thereof, 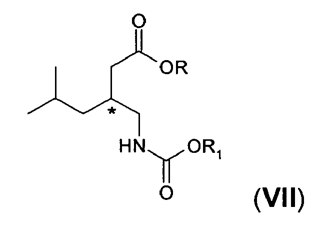 wherein R is C₁-C₁₀ alkyl or aryl-C₁-C₆ alkyl, R₁ is an optionally substituted C₁-C₆ alkyl, aryl or aryl-C₁-C₆ alkyl group, and the asterisk * indicates the presence of a stereogenic carbon.

8. A compound of formula **(VII),** as claimed in claim 7, as the (*S*) enantiomer or a mixture of enantiomers or a salt thereof, selected from:
• Methyl (*S*)-{4-methyl-2-[(1-methoxycarbonyl)-methyl-]-pentyl-}-carbamate,
• Methyl (*S*)-{4-methyl-2-[(1-ethoxycarbonyl)-methyl-]-pentyl-}-carbamate,
• Methyl (*S*)-{4-methyl-2-[(1-propoxycarbonyl)-methyl-]-pentyl-}-carbamate,
• Methyl (*S*)-{4-methyl-2-[(1-butoxycarbonyl)-methyl-]-pentyl-}-carbamate,
• Methyl (*S*)-{4-methyl-2-[(1-isobutyloxycarbonyl)-methyl-]-pentyl-}-carbamate,
• Methyl (*S*)-{4-methyl-2-[(1-octyloxycarbonyl)-methyl-]-pentyl-}-carbamate,
• Ethyl (*S*)-{4-methyl-2-[(1-methoxycarbonyl)-methyl-]-pentyl-}-carbamate,
• Ethyl (*S*)-{4-methyl-2-[(1-ethoxycarbonyl)-methyl-]-pentyl-}-carbamate,
• Ethyl (*S*)-{4-methyl-2-[(1-propoxycarbonyl)-methyl-]-pentyl-}-carbamate,
• Ethyl (*S*)-{4-methyl-2-[(1-butoxycarbonyl)-methyl-]-pentyl-}-carbamate,
• Ethyl (*S*)-{4-methyl-2-[(1-isobutyloxycarbonyl)-methyl-]-pentyl-}-carbamate,
• Ethyl (*S*)-{4-methyl-2-[(1-octyloxycarbonyl)-methyl-]-pentyl-}-carbamate,
• Isopropyl (*S*)-{4-methyl-2-[(1-methoxycarbonyl)-methyl-]-pentyl-}-carbamate,
• Isopropyl (*S*)-{4-methyl-2-[(1-ethoxycarbonyl)-methyl-]-pentyl-}-carbamate,
• Isopropyl (*S*)-{4-methyl-2-[(1-propoxycarbonyl)-methyl-]-pentyl-}-carbamate,
• Isopropyl (*S*)-{4-methyl-2-[(1-butoxycarbonyl)-methyl-]-pentyl-}-carbamate,
• Isopropyl (*S*)-{4-methyl-2-[(1-isobutyloxycarbonyl)-methyl-]-pentyl}-carbamate, and
• Isopropyl (*S*)-{4-methyl-2-[(1-octyloxycarbonyl)-methyl-]-pentyl-}-carbamate.

9. A compound of formula (**VIII**), both as the (*R*) or (*S*) enantiomer, and mixtures thereof or a salt thereof, 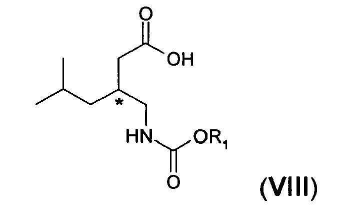 wherein R₁ is an optionally substituted C₁-C₆ alkyl, aryl or aryl-C₁-C₆ alkyl group, and the asterisk * indicates the presence of a stereogenic carbon.

10. A compound of formula **(VIII),** as claimed in claim 9, as the (*R*) or (*S*) enantiomer, or as an enantiomeric mixture thereof or a salt thereof, selected from:
• Methyl {4-methyl-2-[(1-carboxy)-methyl]-pentyl}-carbamate,
• Ethyl {4-methyl-2-[(1-carboxy)-methyl]-pentyl}-carbamate, and
• Isopropyl {4-methyl-2-[(1-carboxy)-methyl]-pentyl}-carbamate.

11. Pregabalin with enantiomeric purity equal to or higher than 99%.

12. Pregabalin with purity higher than 99.5%.
